# EUROPEAN PATENT SPECIFICATION

(11) **EP 2 262 823 B1**
(45) Date of publication and mention of the grant of the patent: **23.01.2013**
(21) Application number: 09718667.0
(22) Date of filing: 12.03.2009
(51) Int. Cl.: C07J 71/00

(54) **PROCESS FOR THE PREPARATION OF PREGNANE DERIVATIVES**
VERFAHREN ZUR HERSTELLUNG VON PREGNANDERIVATEN
PROCÉDÉ POUR LA PRÉPARATION DE DÉRIVÉS DE PRÉGNANE

(30) Priority: 13.03.2008 IT MI20080426; 09.01.2009 IT MI20090016
(43) Date of publication of application: 22.12.2010
(73) Proprietor: Farmabios S.p.A., 27027 Gropello Cairoli (Pavia) (IT)
(72) Inventor: LA LOGGIA, Filippo, I-27027 Gropello Cairoli (PV) (IT); CURTI, Matteo, I-27010 Giussago (PV) (IT); POZZOLI, Claudio Gianluca, I-20052 Monza (IT)
(74) Representative: Longoni, Alessandra
(86) International application number: PCT/EP2009/052945
(87) International publication number: WO 2009/112557

(56) References cited:
- EP-A- 0 875 516
- EP-A- 0 994 119
- WO-A-02/088169
- WO-A-2007/056181
- WO-A-2008/035066

## Description

The present invention relates to a process for the preparation of 16,17-acetals of pregnane derivatives and, more particularly, it relates to a stereoselective and enrichment process for the preparation of compounds of formula 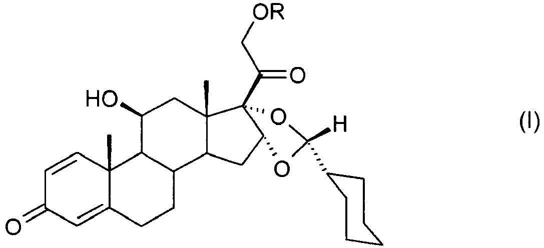 wherein R is hydrogen, acetyl or isobutyryl.

The compound of formula (I) wherein R is isobutyryl is a drug with anti-inflammatory activity, known with the International Non-proprietary Name ciclesonide, used for the treatment of asthma by inhalation. Ciclesonide is the 22R epimer and is a prodrug; the pharmaceutically active form is the compound of formula (I) wherein R is hydrogen.

The processes for the preparation of ciclesonide described in the literature lead to the obtainment of mixtures of epimers R/S which are then enriched by fractional crystallization or chromatographic separation up to reach a content of epimer 22S in compliance with regulatory requirements, that is generally lower than 1%. US5482934 (Elmu SA) and US5733901 (Byk Gulden) disclose the separation of the epimers of ciclesonide or analogs thereof by preparative HPLC.

US5728826 (Byk Gulden) discloses a process for the preparation of ciclesonide comprising the epimeric enrichment of a R/S mixture of silyl derivatives of ciclesonide by fractional crystallization up to obtain a ratio R/S>99/1.

WO98/09982 (Byk Gulden) discloses the epimeric enrichment of ciclesonide by fractional crystallization from a mixture of water and a water-miscible organic solvent, in particular ethanol. Starting from epimeric mixtures R/S=90/10, ciclesonide with an epimeric ratio R/S>99.5/0.5 is obtained after four crystallizations. W02007/056181 (Sicor Inc.) discloses a method to increase the epimeric ratio of ciclesonide by crystallization from a solution of ciclesonide in one or more organic solvent immiscible with water. Four crystallizations are needed to obtain ciclesonide with an epimeric ratio 99.75/0.25 starting from a mixture R/S=90/10.

W02007/092574 (Sicor Inc.) discloses a method for increasing the epimeric ratio R/S of ciclesonide consisting in dissolving ciclesonide in acetone under reflux and adding isooctane while keeping the solution at 90°C and then gradually cooling it. The procedure is repeated four times to obtain ciclesonide with an epimeric ratio R/S=99.75/0.25 starting from a mixture R/S=90/10.

W02008/015696 (Cadila Healthcare Ltd.) discloses a process for the preparation of ciclesonide substantially free from epimer S by chromatographic treatment of a mixture R/S using a chiral stationary phase.

WO02/38584 (Byk Gulden) discloses the preparation of compounds of formula (I) prevailingly as R epimer by transketalization with cyclohexancarboxyaldehyde in the presence of a mineral acid such as tetrafluoroboric acid or perchloric acid or of a sulphonic acid such as methansulphonic acid. The transketalization is carried out without solvents or in the presence of suitable organic solvents such as ethers, esters, halogenated hydrocarbons or nitrohydrocarbons. By working under the same conditions, the ketalization of the 16,17-dihydroxy derivatives of the corresponding compounds of formula (I) leads to the obtainment of the compounds of formula (I) as epimeric mixtures.

US2007/0117974 (Zhejiang Xianju pharmaceutical Co.) discloses an one-pot process for the preparation of ciclesonide starting from 16α-hydroxyprednisolone by reaction with isobutyric anhydride and cyclohexancarboxyaldehyde in the presence of an acid and of a polar organic solvent.

EP 0 875 516 (Farmabios) discloses the preparation of epimeric mixtures of budesonide by acetalization in the presence of aqueous hydrobromic or hydroiodic acid as reaction catalysts and solvents.

EP 0 994 119 (Farmabios) discloses a stereoselective process for the preparation of budesonide in the form of 22R epimer by transketalization in the presence of aqueous hydrobromic or hydroiodic acid as reaction catalysts and solvents.

A simple industrial method for the obtainment of ciclesonide with an epimeric ratio R/S higher then 99/1 has not yet been disclosed in the literature.

We have now surprisingly found that the compounds of formula (I) can be obtained prevailingly in the epimeric form 22R by direct ketalization of 16α-hydroxyprednisolone or of its corresponding acetyl or isobutyryl ester in C21 by reaction with cyclohexancarboxyaldehyde in the presence of aqueous hydrobromic or hydroiodic acid as reaction catalysts and solvents and, optionally, obtained in the substantially pure epimeric form 22R by enrichment of an epimeric mixture R/S of a compound of formula

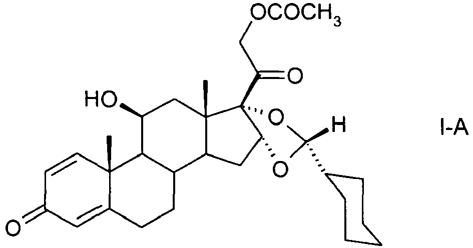

Therefore, object of the present invention is a stereoselective process for the preparation of the compounds of formula (I) comprising the ketalization of 16α-hydroxyprednisolone or of its acetyl or isobutyryl ester in C21 by reaction with cyclohexancarboxyaldehyde in the presence of aqueous hydrobromic or hydroiodic acid as reaction catalysts and solvents.

The process object of the present invention allows to obtain the compounds of formula (I) prevailingly as 22R epimer.

In the present context, unless otherwise specified, the expression "prevailingly as 22R epimer" means that the compounds of formula (I) have an epimeric ratio 22R/22S higher than 90/10, preferably higher or equal to 95/5, and the expression "in substantially pure epimeric form R" or similar means a compound with an epimeric ratio R/S>99/1.

In the process object of the present invention the aqueous hydrobromic or hydroiodic acid is used at concentrations from about 20% to about 70% by weight. Mainly for practical reasons, aqueous hydrobromic acid at concentrations from about 48% to about 62% by weight, generally about 48%, or aqueous hydroiodic acid at concentrations from about 56% to about 67% by weight, generally about 55-57%, are used since they are already commercially available.

The amount of aqueous hydrobromic or hydroiodic acid used in the process is generally from 1 to 20 parts by volume, preferably about 10 parts by volume, per part of the starting 16,17-dihydroxy compound. The amount of cyclohexancarboxyaldehyde is generally from 0.2 to 1 mole per mole of starting 16,17-dihydroxy compound.

The ketalization according to the process of the present invention is generally carried out at a temperature from -10°C and +30°C, preferably from about -2°C to about +2°C.

The reaction time is generally short, preferably shorter than 10 hours.

In a practical preferred embodiment, the stereoselective process of the present invention allows to obtain ciclesonide prevailingly in form of epimer 22R.

For the preparation of ciclesonide, the ketalization reaction according to the present invention can be carried out starting from 16α-hydroxyprednisolone or starting from the corresponding ester. Ciclesonide is preferably prepared by ketalization of 16α-hydroxyprednisolone with cyclohexancarboxyaldehyde, followed by esterification of the resultant ketal with a reactive derivative of isobutyric acid, preferably isobutyric anhydride, according to known methods. At the end of the esterification reaction, the epimeric ratio of the starting ketal remains substantially unchanged in the resultant ciclesonide.

The compounds of formula (I) with epimeric ratios higher than 90:10 can be purified according to conventional crystallization techniques to obtain the 22R epimer in substantially pure form.

In a more preferred embodiment, ciclesonide is prepared by ketalization of 16α-hydroxyprednisolone with cyclohexancarboxyaldehyde, followed by esterification of the resultant ketal with a reactive derivative of acetic acid, preferably acetic anhydride, according to known methods. The resultant compound of formula I-A, prevailingly in the epimeric form R, is treated with methanol to obtain the compound of formula I-A in substantially pure epimeric form R. After hydrolysis of the acetyl group and subsequent esterification with a reactive derivative of isobutyric acid, preferably isobutyric anhydride, ciclesonide in substantially pure epimeric form is obtained.

A further preferred object of the present invention is therefore a process for the preparation of ciclesonide in substantially pure form 22R comprising:
(i) the ketalization of 16α-hydroxyprednisolone by reaction with cyclohexancarboxyaldheyde in the presence of aqueous hydrobromic or hydroiodic acid as reaction catalysts and solvents; and,
(ii) the treatment of the resultant epimeric mixture R/S of the compound of formula I-A with methanol, followed by the isolation of the compound of formula I-A in substantially pure epimeric form R
(iii) the conversion of the compound of formula I-A into ciclesonide by hydrolysis and subsequent esterification with a reactive derivative of isobutyric acid.

The compound of formula I-A is known and was described in the already cited US5482934. Its use as intermediate for the preparation of ciclesonide has been never disclosed in the literature and is a further object of the present invention.

The more advantageous and preferred feature of the process object of the present invention is the possibility to obtain a mixture R/S of compounds of formula (I), preferably the compound I-A, prevailingly in form R. When the compound of formula I-A is obtained, the epimeric mixture R/S can be enriched by simple treatment with methanol to obtain the compound of formula I-A in substantially pure epimeric form R.

The treatment with methanol consists in the suspension of the compound of formula I-A in methanol under stirring at a temperature from 15°C to 35°C, preferably from 20°C to 25°C for 0.5-3 hours.

The suspension can be prepared by directly treating the solid compound of formula I-A with methanol: alternatively, the suspension can be prepared starting from a concentrated solution of the compound I-A in an organic solvent in which the compound I-A is soluble, preferably a halogenated hydrocarbon, still more preferably methylene chloride, by adding methanol and removing the organic solvent. Moreover, the suspension of the compound of formula I-A, which should be enriched in its epimer R content, can be obtained by concentration of the methanolic mother liquors coming from previous enrichment treatment according to the present invention.

With the enrichment process of the present invention the compound I-A in substantially pure epimeric form R can be obtained after only two treatments with methanol starting from an epimeric mixture R/S=90/10. The same result can be obtained after only three treatments starting from an epimeric mixture R/S=85/15. The high efficiency of the enrichment process according to the present invention allows to enrich also epimeric mixtures R/S≤1/1, for example epimeric mixtures contained in the mother liquors of a previous enrichment treatment.

As said above, the compound I-A in substantially pure epimeric form R is then converted into ciclesonide having the same epimeric purity, preferably by alkaline hydrolysis of the acetoxy group and subsequent re-esteriifcation with a reactive derivative of isobutyric acid.

The very high yields of the hydrolysis and re-esterification reactions, together with the preservation of the epimeric purity, make the process object of the present invention particularly advantageous form the industrial point of view with respect to the known methods.

According to our experimental observations, the epimeric enrichment phase by treatment with methanol, object of the present invention, proceeds through the formation of a hemimethanolate of the compound I-A (16α,17-cyclohexylmethylendioxy-11β-hydroxy-21-acetoxypregna-1,4-diene-3,20-dione).

From a practical point of view, however, the isolation of the hemimethanolate of the compound I-A is not necessary to obtain the desired enrichment in epimer R according to the process object of the present invention.

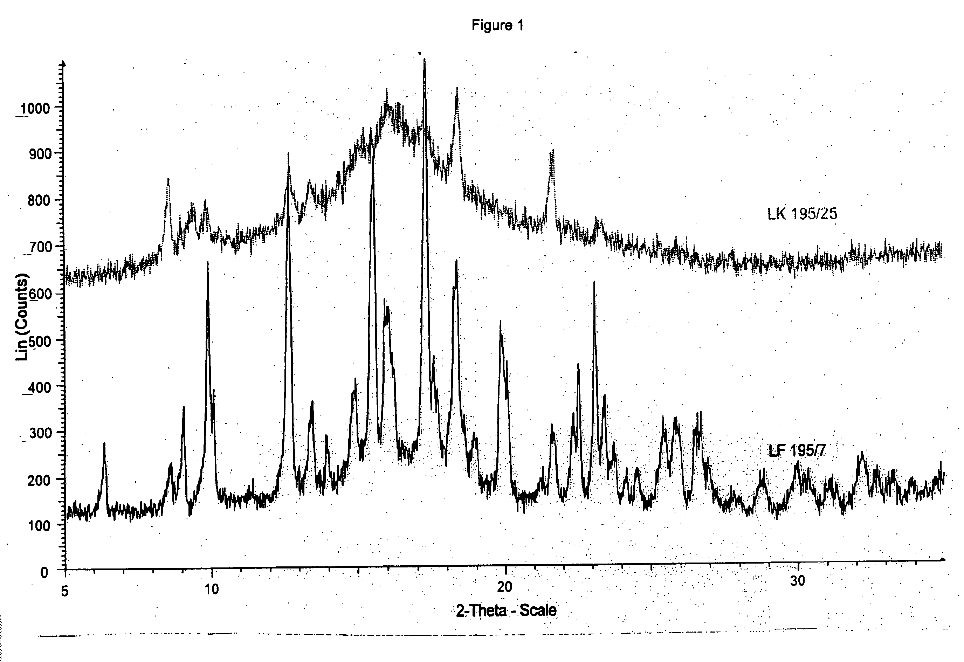

Figure 1 shows the diffraction profiles (XRD) of the hemimethanolate of 16α,17-cyclohesylmethylendioxy-11-hydroxy-21-acetoxy-pregna-1,4-diene-3,20-dione (LF 195/7) and of the corresponding unsolvate compound (LK195/25).

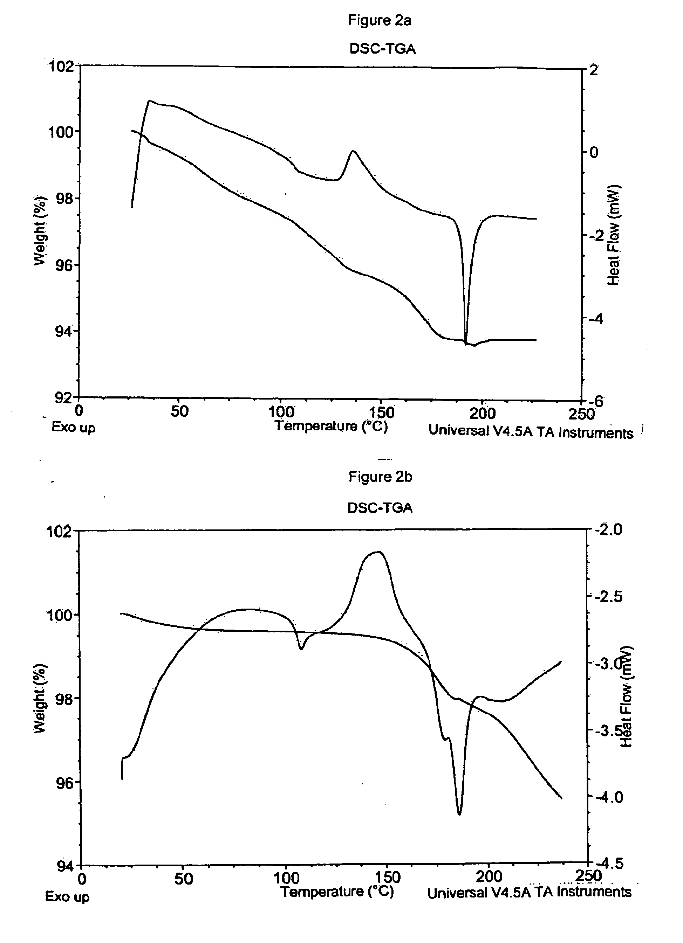

Figures 2a and 2b show the thermogravimetric (TGA) and calorimetric (DSC) analysis of the 16α,17-cyclohesylmethylendioxy-11β-hydroxy-21-acetoxy-pregna-1,4-diene-3,20-dione hemimethanolate and of the corresponding unsolvate compound, respectively.

In order to better illustrate the present invention, without limiting it, the following examples are now given.

### Example 1

### Preparation of 16α,17-cyclohexylmethylendioxy-11β,21-dihydroxypregna-1,4-diene-3,20-dione

Cyclohexancarboxyaldehyde (0.4 parts) was slowly added to 48% hydrobromic acid (10 parts) at 0°C. The reaction mixture was cooled again to 0°C and in a short time 16α-hydroxyprednisolone was added. The mixture was kept at 0°C ÷ +2°C for 60 minutes and then further cyclohexancarboxyaldehyde (0.6 parts) was added raising the temperature to 20°C.

After 2 hours, the mixture was poured into iced purified water (50 parts) and kept under stirring at a temperature lower than +10°C for about 1 hour. The resultant suspension was filtered and the solid was washed with purified water (40 parts) up to neutrality. The epimeric purity of the resultant solid was determined by HPLC obtaining a value of the epimeric ratio 22R:22S=94.6:5.4.

### Example 2

### Preparation of 16α,17-cyclohexylmethylendioxy-11β,21-dihydroxypregna-1,4-diene-3,20-dione

16-Hydroxyprednisolone was first added to a solution of 48% hydrobromic acid (10 parts) cooled at 0°C/+2°C and subsequently cyclohexancarboxyaldehyde (0.42 parts) was slowly added. The reaction mixture was kept under stirring at 0°C/+2°C up to complete conversion (15 hours).

The mixture was poured into iced purified water (50 parts). After about 1 hour under stirring at +5°C/+10°C, the solid was filtered and washed with purified water up to neutrality. The wet product was charged into a reactor containing a mixture of methanol (7.5 parts) and purified water (0.75 parts). The mixture was heated under reflux for about 1 hour, then was cooled up to room temperature. After further cooling at about +5°C, the solid was filtered, washed with a mixture water/methanol=3/1 (4 parts) and dried under vacuum at +60°C.
Yield w/w = 112-120% (theoretical 125%)
Epimeric ratio R/S=90.3/9.7

### Example 3

### Preparation of 16α, 17-cyclohexylmethylendioxy-11β-hydroxy-21-acetoxypregna-1,4-diene-3,20-dione

Pyridine (5 parts) and 16α,17-cyclohexylmethylendioxy-11β,21-dihydroxypregna-1,4-diene-3,20-dione, prepared as described in example 2, were charged into a reactor. After cooling at 0°C/+2°C, acetic anhydride (5 parts) was slowly added. The reaction mixture was kept under stirring at 0°C/+2°C up to complete conversion (15 hours).

Ice was added to the reaction mixture. After about 1 hour, the reaction mixture was poured into iced water (45 parts) and 50% sulphuric acid (5 parts). The mixture was kept under stirring at +5°C/+10°C for about 1 hour, then the solid was filtered and washed with purified water up to neutrality obtaining crude 16α,17-cyclohexylmethylendioxy-11β-hydroxy-21-acetoxypregna-1,4-diene-3,20-dione with epimeric ratio R/S=90.4/9.6.

### Enrichment in 22R epimer

Wet 16α,17-cyclohexylmethylendioxy-11β-hydroxy-21-acetoxypregna-1,4-diene-3,20-dione (R/S=90.4/9.6), obtained as previously described, was dissolved in dichloromethane (5 parts). After separation of the layers, water was separated and the organic layer was concentrated under vacuum up to an oil. Methanol (about 30 parts) was then added and the mixture was heated under reflux by distilling off the eventual still present dichloromethane. After slow cooling to room temperature, the precipitate was filtered and washed with iced methanol.

The resultant product can be treated again as previously described or, more simply treated with methanol (12 parts) at room temperature. In both cases (recrystallization or simple trituration from alcohol) the compound of formula I-A was obtained with high purity (HPLC >>99%) and with epimeric ratio 22R/22S as reported in the following table

| | epimer R | epimer S |
|---|---|---|
| First crop | 97% | 3% |
| Second crop | >99% | <1% |
| Triturated | >99% | <1% |

Yield w/w = 80-85% (theoretical 109%)

### Example 4

### Preparation of (22R)-16α,17-cyclohexylmethylendioxy-11β,21-dihydroxypregna-1,4-diene-3,20-dione

### Procedure A

(22R)-16α,17-cyclohexylmethylendioxy-11β-hydroxy-21-acetoxypregna-1,4-diene-3,20-dione, prepared as described in example 3, dichloromethane (5 parts) and methanol (7.5 parts) were charged into a reactor. The mixture was cooled at 0°C/+2°C, then 30% sodium hydroxide (0.175 parts) and water (1.75 parts) were slowly added. The reaction mixture was kept under stirring at 0°C/+2°C up to complete conversion (1 hour).

Acetic acid 80% (0.09 parts) was added to the reaction mixture. After clarification by filtration, the resultant mixture was concentrated under vacuum up to complete removal of dichloromethane. At warm, water (7 parts) was added and methanol was removed by distillation. After cooling to room temperature, the precipitate was isolated by filtration and washed with water. The product was dried under vacuum at 70°C for 14 hours.

| | epimer R | epimer S |
|---|---|---|
| Isolated solid | >99% | <1% |

Yield w/w = 75-80% (theoretical 91.8%)

### Example 5

### Preparation of ciclesonide

(22R)-16α,17-cyclohexylmethylendioxy-11β,21-dihydroxypregna-1,4-diene-3,20-dione, prepared as described in example 4, was dissolved in pyridine (5 parts) under stirring at room temperature. After cooling at 0-2°C, isobutyric anhydride (0.5 parts) was slowly added and the reaction mixture was kept at room temperature for about 15 hours.

When the reaction was complete, ice (1 part) was added. The mixture was kept under stirring for about 30 minutes, then was slowly poured under stirring into iced purified water (45 parts) and 50% sulphuric acid (5 parts). After about 2 hours, the crystallized product was filtered and washed with water up to neutrality. The resultant wet product was charged again into a reactor and added with acetone (4 parts). After clarification, the solution was slowly poured into water cooled at 10-15°C. After about 1 hour under stirring, the solid was filtered, washed with water and dried in oven under vacuum at about +70°C for 14 hours.
Yield w/w = 110%; HPLC purity > 99.7 (sum of 22R and 22S)

| Isomer R | Isomer S |
|---|---|
| >99% | <1% |

### Example 6

### Enrichment in 22R epimer

An epimeric mixture R/S=83/17 of 16a,17-cyclohexylmethylendioxy-11β-hydroxy-21-acetoxypregna-1,4-diene-3,20-dione (4.3 g) was charged into a flask with methanol (86 ml) under stirring. The suspension was heated at 50°C and kept at this temperature for 15 minutes. After cooling at room temperature, the suspension was kept under stirring for about 2.5 hours. The crystalline solid was filtered, washed with methanol (8.6 ml) and dried in oven under vacuum at 70°C for about 4 hours and then at about 55°C for 48 hours obtaining an epimeric mixture with an epimer R content equal to 97.8% (3.2 g; yield 74.4%).

### Example 7

### Preparation of 16α,17-cyclohexylmethylendioxy-11β-hydroxy-21-acetoxypregna-1,4-diene-3,20-dione

Pyridine (45 ml) and 16α,17-cyclohexylmethylendioxy-11β,21-dihydroxypregna-1,4-diene-3,20-dione (9 g; ratio R/S=85.8/14.2) were charged into a flask under stirring and under nitrogen. After cooling at 0°C/+2°C, acetic anhydride (4.5 ml) was added to the solution. The reaction mixture was then kept under stirring at room temperature up to complete conversion (5 hours).

After addition of ice, the reaction mixture was kept under stirring for 30 minutes and then poured into iced water (405 ml) and 50% sulphuric acid (45 ml). The mixture was kept under stirring for about 1.5 hours, then the solid was filtered and washed with purified water up to neutrality obtaining crude 16α,17-cyclohexylmethylendioxy-11β-hydroxy-21-acetoxypregna-1,4-diene-3,20-dione with an epimer R content equal to 85.3%.

The crude was dissolved in dichloromethane (90 ml) and added with 50% sulphuric acid up to pH 3-4. After separation of the layers, water was separated and the organic phase was charged into a 10% NaCl solution keeping under stirring at room temperature for 15 minutes. The phases were separated again and methanol was added to the organic phase then isolating by filtration a crystalline solid (8.2 g) with an epimer R content = 95.8%.

The resultant crystalline product was charged into methanol (135 ml), keeping the suspension under mild stirring for about 30 minutes. After cooling at 15°C, the solid was filtered, washed with iced methanol (18 ml) and dried in oven under vacuum at 70°C obtaining 16α,17-cyclohexylmethylendioxy-11β-hydroxy-21-acetoxypregna-1,4-diene-3,20-dione (7 g; yield 77.7% - ratio R/S=99/1).

The methanolic mother liquors containing 16α,17-cyclohexylmethylendioxy-11β-hydroxy-21-acetoxypregna-1,4-diene-3,20-dione with an epimeric ratio R/S=1/1 were concentrated up to a volume of about 50 ml and then kept under stirring at room temperature. After about 15 minutes the crystallization began. The suspension was kept under stirring at room temperature for about 90 minutes. The solid was filtered, washed with iced methanol (7 ml) and dried in oven under vacuum at 70°C obtaining 16α,17-cyclohexylmethylendioxy-11β-hydroxy-21-acetoxypregna-1,4-diene-3,20-dione (0.77 g) with epimeric ratio R/S=83/17.

### Example 8

### Isolation of 16α,17-cyclohexylmethylendioxy-11β-hydroxy-21-acetoxvpregna-1,4-diene-3,20-dione hemimethanolate

Methylene chloride (227.5 ml), methanol (45.5 ml) and 16α,17-cyclohexylmethylendioxy-11β-hydroxy-21-acetoxypregna-1,4-diene-3,20-dione (45.5 g; ratio R/S=96.8/3.2) were charged into a flask. The suspension was heated under mild reflux up to obtain a solution which was then concentrated under atmospheric pressure up to a volume of 90-100 ml. After dilution with methanol (227.5 ml), the mixture was concentrated again under atmospheric pressure up to a steam temperature of 64-65°C and a final internal volume of about 125-135 ml. The crystalline product was cooled at 20°C and kept at that temperature for about 1 hour. After filtration and washing with iced methanol (90 ml), the solid was dried in oven under vacuum at 70°C overnight obtaining 16α,17-cyclohexylmethylendioxy-11β-hydroxy-21-acetoxypregna-1,4-diene-3,20-dione hemimethanolate (44.1 g; yield 96.9%; ratio R/S=99.3/0.7).

## Claims

1. A stereoselective process for the preparation of the compounds of formula (I) 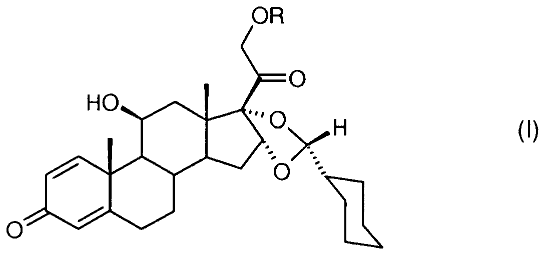 wherein R is hydrogen, acetyl or isobutyryl;
comprising the ketalization of 16α-hydroxyprednisolone or of its acetyl or isobutyryl ester in C21 by reaction with cyclohexancarboxyaldehyde in the presence of aqueous hydrobromic or hydroiodic acid as reaction catalysts and solvents.

2. A process according to claim 1 wherein the compounds of formula (I) are obtained with an epimeric ratio 22R/22S higher or equal to 95/5.

3. A process according to claim 1 for the preparation of ciclesonide in substantially pure form 22R comprising:
(i) the ketalization of 16α-hydroxyprednisolone by reaction with cyclohexancarboxyaldheyde in the presence of aqueous hydrobromic or hydroiodic acid as reaction catalysts and solvents followed by esterification of the resultant ketal with a reactive derivative of acetic acid; and,
(ii) the treatment of the epimeric mixture R/S of the compound of formula I-A 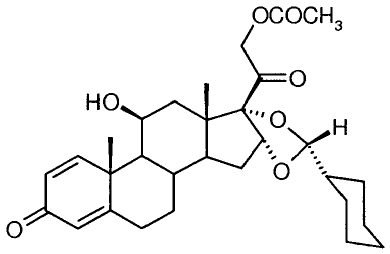 with methanol, followed by the isolation of the compound of formula I-A in substantially pure epimeric form R
(iii) the conversion of the compound of formula I-A into ciclesonide by hydrolysis and subsequent esterification with a reactive derivative of isobutyric acid.

4. A process according to claim 1 wherein aqueous hydrobromic or hydroiodic acid is used at concentrations from about 20% to about 70% by weight.

5. A process according to claim 1 wherein the amount of aqueous hydrobromic or hydroiodic acid used in the process is from 1 to 20 parts by volume per part of the starting 16,17-dihydroxy compound.

6. A process according to claim 1 wherein the amount of cyclohexancarboxyaldehyde is from 0.2 to 1 mole per mole of the starting 16,17-dihydroxy compound.

7. A process according to claim 1 for the preparation of ciclesonide.

8. A process according to claim 3 wherein the treatment with methanol consists in suspending the compound of formula I-A in methanol under stirring at a temperature from 15°C and 35°C.

9. A process according to claim 8 wherein the suspension is prepared by directly treating the solid compound of formula I-A with methanol.

10. A process according to claim 8 wherein the suspension is prepared from a concentrated solution of the compound of formula I-A in an organic solvent in which the compound of formula I-A is soluble.

## Patentansprüche

1. Stereoselektiver Verfahren zur Herstellung der Verbindungen mit der Formel (I) 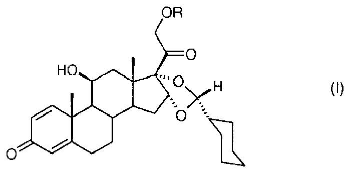 wobei R Wasserstoff, Acetyl oder Isobutyryl ist;
der die Ketalisierung von 16α-Hydroxyprednisolon oder dessen Acetyls oder Isobutyrylesters in C21 mittels Reaktion mit Cyclohexancarboxyaldehyd in Gegenwart von wässriger Bromwasserstoff- oder lodwasserstoffsäure als Reaktionskatalysatoren und Lösungsmitteln umfasst.

2. Verfahren nach Anspruch 1, wobei die Verbindungen mit der Formel (I) mit einem Epimer-Verhältnis von 22R:22S erhalten werden, das höher oder gleich 95:5 ist.

3. Verfahren nach Anspruch 1 zur Herstellung von Ciclesonid in im Wesentlichen reiner Form 22R, der Folgendes umfasst:
(i) Ketalisierung von 16α-Hydroxyprednisolon mittels Reaktion mit Cyclohexancarboxyaldehyd in Gegenwart von wässriger Bromwasserstoff- oder lodwasserstoffsäure als Reaktionskatalysatoren und Lösungsmitteln, gefolgt von der Veresterung des sich ergebenden Ketals mit einem reaktiven Derivat von Essigsäure; und
(ii) Behandlung der Epimer-Mischung R/S der Verbindung mit der Formel I-A 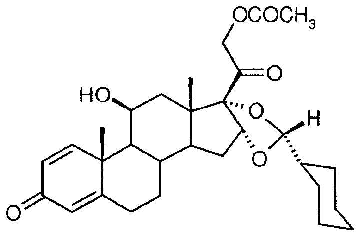 mit Methanol, gefolgt von der Isolierung der Verbindung mit der Formel I-A in im Wesentlichen reiner Epimer-Form R
(iii) Umwandlung der Verbindung mit der Formel I-A in Ciclesonid mittels Hydrolyse und nachfolgende Veresterung mit einem reaktiven Derivat von Isobuttersäure.

4. Verfahren nach Anspruch 1, wobei wässrige Bromwasserstoffsäure oder lodwasserstoffsäure in Konzentrationen von etwa 20 Gew.-% bis etwa 70 Gew.- % verwendet wird.

5. Verfahren nach Anspruch 1, wobei die Menge der in dem Verfahren verwendeten wässrigen Bromwasserstoffsäure oder lodwasserstoffsäure 1 bis 20 Volumenteile pro Teil der 16,17-Dihydroxy-Ausgangsverbindung beträgt.

6. Verfahren nach Anspruch 1, wobei die Menge des Cyclohexancarboxyaldehyds 0,2 bis 1 Mol pro Mol der 16,17-Dihydroxy-Ausgangsverbindung beträgt.

7. Verfahren nach Anspruch 1 zur Herstellung von Ciclesonid.

8. Verfahren nach Anspruch 3, wobei die Behandlung mit Methanol darin besteht, dass die Verbindung mit der Formel I-A unter Rühren bei einer Temperatur von 15°C und 35°C in Methanol suspendiert wird.

9. Verfahren nach Anspruch 8, wobei die Suspension durch direkte Behandlung der festen Verbindung mit der Formel I-A mit Methanol hergestellt wird.

10. Verfahren nach Anspruch 8, wobei die Suspension aus einer konzentrierten Lösung der Verbindung mit der Formel I-A in einem organischen Lösungsmittel hergestellt wird, in dem die Verbindung mit der Formel I-A löslich ist.

## Revendications

1. Procédé stéréosélectif pour la préparation des
composés de formule (I) : 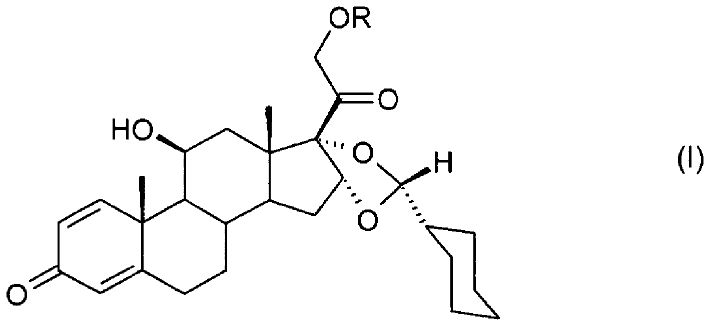 dans lequel R est l'hydrogène, un groupement acétyle ou isobutyryle,
comprenant la cétalisation du 16α-hydroxyprednisolone ou de son ester d'acétyle ou d'isobutyryle en C₂₁ par réaction avec du cyclohexanecarboxyaldéhyde en présence d'acide bromhydrique ou iodhydrique aqueux comme catalyseurs et solvants de la réaction.

2. Procédé selon la revendication 1, dans lequel les composés de formule (I) sont obtenus avec un rapport épimère 22R/22S supérieur ou égal à 95/5.

3. Procédé selon la revendication 1 pour la préparation de ciclésonide sous forme sensiblement pure de 22R comprenant :
i) la cétalisation du 16α-hydroxyprednisolone par réaction avec du cyclohexanecarboxyaldéhyde en présence d'acide bromhydrique ou iodhydrique aqueux comme catalyseurs et solvants de la réaction, puis l'estérification du cétal obtenu avec un dérivé réactif de l'acide acétique ; et
ii) le traitement du mélange épimère R/S du composé de formule (I-A) : 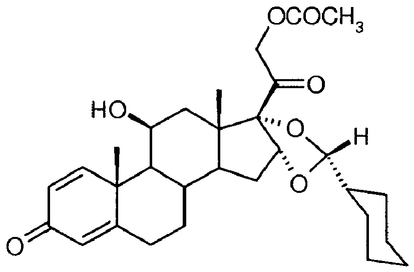 avec du méthanol, puis l'isolement du composé de formule (I-A) sous forme épimère sensiblement pure R ;
iii) la conversion du composé de formule I-A en ciclésonide par hydrolyse et estérification ultérieure avec un dérivé réactif de l'acide isobutyrique.

4. Procédé selon la revendication 1, dans lequel l'acide bromhydrique ou iodhydrique aqueux est utilisé en concentrations d'environ 20 à environ 70 % en poids.

5. Procédé selon la revendication 1, dans lequel la quantité d'acide bromhydrique ou iodhydrique aqueux utilisée dans le procédé est de 1 à 20 parties en volume par partie du composé 16,17-dihydroxylé de départ.

6. Procédé selon la revendication 1, dans lequel la quantité de cyclohexanecarboxyaldéhyde est de 0,2 à 1 mole par mole du composé 16,17-dihydroxylé de départ.

7. Procédé selon la revendication 1 pour la préparation de ciclésonide.

8. Procédé selon la revendication 3, dans lequel le traitement au méthanol consiste à mettre en suspension le composé de formule I-A dans du méthanol sous agitation à une température de 15 à 35 °C.

9. Procédé selon la revendication 8, dans lequel la suspension est préparée en traitant directement le composé solide de formule I-A au méthanol.

10. Procédé selon la revendication 8, dans lequel la suspension est préparée à partir d'une solution concentrée du composé de formule I-A dans un solvant organique dans lequel le composé de formule I-A est soluble.
